# EUROPÄISCHE PATENTSCHRIFT

(11) **EP 1 600 444 B1**
(45) Veröffentlichungstag und Bekanntmachung des Hinweises auf die Patenterteilung: **30.04.2008**
(21) Anmeldenummer: 05009939.9
(22) Anmeldetag: 06.05.2005
(51) Int. Cl.: C07D 233/54

(54) **Verfahren zur Herstellung von N,N'-Carbonyldiazolen**
Process for the preparation of N,N'-Carbonyldiazoles
Procédé pour la préparation de N,N'-Carbonyldiazoles

(30) Priorität: 13.05.2004 DE 102004023606
(43) Veröffentlichungstag der Anmeldung: 30.11.2005
(73) Patentinhaber: Saltigo GmbH, 40764 Langenfeld (DE)
(72) Erfinder: Job, Andreas, Dr., 50858 Köln (DE); Griehsel, Bernd, Dipl.-Ing., 46242 Bottrop (DE)
(74) Vertreter: Wichmann, Birgid

(56) Entgegenhaltungen:
- EP-A- 0 692 476
- WO-A-00/06551
- WO-A-00/14072

## Beschreibung

Die vorliegende Erfindung betrifft ein verbessertes Verfahren zur Herstellung von N,N'-Carbonyldiazolen durch Umsetzung von Azolen mit Phosgen.

Es ist bereits bekannt, dass man N,N'-Carbonyldiazole erhalten kann, indem man Azole mit Phosgen umsetzt (DE-AS 10 33 210 und Liebigs Ann. Chem. **1957**, *609*, 75). Dabei wird ausschließlich wasserfreies Tetrahydrofuran in den beschriebenen Beispielen eingesetzt, wobei eine Lösung des gesamten Azols in wasserfreiem Tetrahydrofuran vorgelegt und dann das Phosgen eingeleitet wird. Die Umsetzung erfolgt bei Raumtemperatur. Auffällig ist die geringe Konzentration des Azols in THF als Lösungsmittel von 2 bis 4 Gew.-%. In DE-AS 1 033 210 wird nur allgemein und ohne jeglichen Beleg ausgeführt, dass anstelle von Tetrahydrofuran prinzipiell auch andere Ether oder aliphatische oder aromatische Kohlenwasserstoffe als Lösungsmittel einsetzbar seien. In einem in Chem. Ber. 1963, 96, 3374 beschriebenen Verfahren werden bei analoger Vorlage des gesamten Azols in einem THF-Benzol-Lösungsmittelgemisch ebenfalls nur Konzentrationen von etwa 7 Gew.-% des Azols im Lösungsmittelgemisch erreicht.

Nach einem neueren Verfahren gemäß EP-A-692 476 können in aromatischen Lösungsmitteln wie Benzol, Toluol, Xylolen, Chlorbenzolen oder Gemischen davon, die vor der Umsetzung jeweils durch Andestillieren entwässert werden, bei Temperaturen von 50 bis 120°C etwas höhere Konzentrationen des Azols im Lösungsmittel erreicht werden. Beschrieben werden Konzentrationen im Bereich bis zu 12 Gew.-%. Hierbei wird zunächst das Lösungsmittel durch Andestillieren entwässert, dann das Azol zugegeben, dieses unter Erwärmung gelöst und dann Phosgen eingeleitet.

In der WO-A-00/14072 ist ein Verfahren zur Herstellung von Carbonyldiimidazol aus Imidazol und Phosgen bei einer Temperatur von 60 bis 80°C beschrieben, welches in ortho-, meta- oder para-Xylol, Mischungen daraus oder in Chlorbenzol als Lösungsmittel durchgeführt wird und bei dem das als Koppelprodukt anfallende Imidazol-Hydrochlorid als Schmelze durch Phasentrennung aus der erhaltenen Reaktionsmischung bei einer Temperatur größer 100°C abgetrennt wird. Die Umsetzung als solche wird durchgeführt, indem man Phosgen zu dem in Lösung vorgelegten Imidazol zudosiert.

Aus der DE-A-198 33 913 ist ein Verfahren zur Herstellung von N,N'-Carbonyldiazolen bekannt, bei dem unter Verwendung aromatischer Lösungsmittel wie Benzol, Toluol, Xylol oder chlorierten Benzolen gearbeitet wird, die vorab durch Andestillieren entwässert werden. Wesentlich ist bei diesem Verfahren, dass das Azol, gelöst in einem der genannten aromatischen Lösungsmittel, und Phosgen parallel in weiteres vorgelegtes Lösungsmittel zudosiert werden. Es wird beschrieben, dass durch diese Art der Verfahrensführung eine Konzentration des Azols von bis zu 33 Gew.-% erreichbar ist. Da aber ein Teil des verwendeten Lösungsmittels mit den Reaktanden zugegeben wird, entfällt hier die Möglichkeit, das gesamte Lösungsmittelvolumen in dem für die Reaktion vorgesehenen Kessel azeotrop zu trocknen. Eine Parallel-Dosierung ist jedoch prinzipiell eine Technik, die die wirtschaftliche Attraktivität eines Verfahrens verringert. Im konkreten Fall ist eine vernünftige Filtration des als Nebenprodukt entstehenden Azol-Hydrochlorids nur gegeben, wenn ein ganz bestimmtes Dosierverhältnis exakt eingehalten wird. Das Verfahren zeigt darüber hinaus eine Empfindlichkeit gegen Überphosgenierung, welche zu einer Dunkelfärbung des erhaltenen Produkts führt.

Sowohl bei dem Verfahren gemäß DE-A-198 33 913 als auch den übrigen zuvor beschriebenen Verfahren, die aromatische Lösungsmittel verwenden und bei Temperaturen von über 50°C ablaufen, besteht die Gefahr, dass der bei der Reaktion entstehende Azol-Hydrochlorid-Niederschlag als zähe, klebrige Masse anfällt. Diese haftet fest an Gefäßwand und Rührer, wodurch die Rührbarkeit des Systems sehr erschwert wird. Die Schwergängigkeit des Rührens begrenzt die maximal mögliche Raum-Zeit-Ausbeute auf sehr geringe Werte. Bei der Verfestigung des Niederschlags gegen Ende der Phosgenzugabe entstehen harte Kugeln, die zudem am Reaktionsgefäß und seinen Einbauten (z.B. Rührer, Tauchrohre etc.) Beschädigungen verursachen können.

Es besteht deshalb das Bedürfnis nach einem verbesserten Verfahren zur Herstellung von N,N'-Carbonyldiazolen mit einer möglichst einfachen Verfahrensführung, bei dem keine klebrigen und Probleme verursachenden Azol-Hydrochlorid-Niederschläge auftreten und ferner keine hohen Reaktionstemperaturen erforderlich sind, welche die Wirtschaftlichkeit des Verfahrens durch hohe Energiekosten verringern würden.

Gegenstand der Erfindung ist ein Verfahren zur Herstellung von N,N'-Carbonyldiazolen der allgemeinen Formel (I) 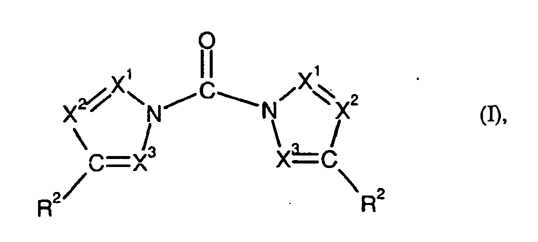 wobei entweder
- X¹, X² und X³: unabhängig voneinander jeweils für CR¹ oder Stickstoff stehen, wobei R¹ Wasserstoff oder geradkettiges oder verzweigtes C₁-C₆-Alkyl bedeutet und
- R²: Wasserstoff bedeutet,
oder
- X¹ und X³: für CR¹ stehen, wobei der in X¹ befindliche Rest R¹ Wasserstoff oder geradkettiges oder verzweigtes C₁-C₆-Alkyl bedeutet und der in X³ befindliche Rest R¹ gemeinsam mit R² eine -CH=CH-CH=CH-Brücke bildet, und
- X²: für CR¹ oder Stickstoff steht, wobei R¹ Wasserstoff oder geradkettiges oder verzweigtes C₁-C₆-Alkyl bedeutet,
durch Umsetzung von Azolen der allgemeinen Formel (II), 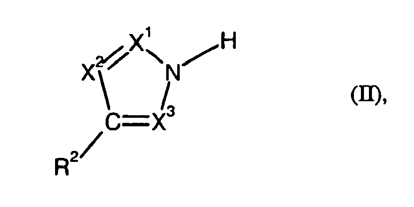 in der die verwendeten Reste und Symbole die für die allgemeine Formel (I) angegebenen Bedeutungen haben,
mit Phosgen, wobei dieses Verfahren dadurch gekennzeichnet ist, dass
(i) ein halogeniertes aliphatisches Lösungsmittel aus der Gruppe der chlorierten aliphatischen Kohlenwasserstoffe, der bromierten aliphatischen Kohlenwasserstoffe und der gemischt Chlor-Brom-substituierten aliphatischen Kohlenwasserstoffe verwendet wird und
(ii) dass man die gesamte umzusetzende Menge Azol vorlegt und anschließend Phosgen eindosiert.

Beim erfindungsgemäßen Verfahren entsteht und verbleibt das bei der Reaktion ausfallende Azol-Hydrochlorid, wie z.B. das Imidazol-Hydrochlorid, im Gegensatz zu den in der EP-A-692 476, DE-A-198 33 913 und WO-A-00/14072 beschriebenen Verfahren immer als gut rührbarer, kristalliner Niederschlag, der keine Anbackungen und Verklebungen an Rührer oder Gefäßwand bildet. Durch den dispersen Habitus des Niederschlags ist der Rührwiderstand deutlich geringer als bei nicht erfindungsgemäßer Arbeitsweise. Überraschenderweise kann man erfindungsgemäß deutlich höhere Reaktanden-Konzentrationen als bei den bisher bekannten Verfahren mit vergleichbarer nicht-paralleler Dosierweise erzielen, was eine deutlich verbesserte Raum-Zeit-Ausbeute gegenüber dem Stand der Technik zur Folge hat: Bei den Verfahren der EP-A-692476 und WO-A-00/14072 wird eine Reaktandenkonzentration von 11% bzw. 11,9 Gew.-% in dem jeweiligen Lösungsmittel angegeben, während man mit dem erfindungsgemäßen Verfahren Reaktandenkonzentrationen von bis zu 25 Gew.-% erhält. Auch können Beschädigungen des Reaktors und seiner Einbauten durch harte Azol-Hydrochlorid-Konglomerate ausgeschlossen werden. Darüber hinaus besitzt das erfindungsgemäße Verfahren nur eine sehr geringe Empfindlichkeit gegenüber einer Überphosgenierung, die bei anderen Verfahren zu Verfärbungen des isolierten Produkts führt. Die Qualität der so erhaltenen N,N'-Carbonyldiazole zeichnet sich somit insbesondere durch eine sehr gute Farbzahl nach Hazen aus.

Im erfindungsgemäßen Verfahren können entweder zwei verschiedene oder aber auch nur ein einziges Azol der allgemeinen Formel (II) eingesetzt werden. Im ersten Fall wird ein N,N'-Carbonyl-diazol der Formel (I) erhalten, bei dem die beiden Azolringe verschieden sind. Im zweiten Fall entsteht ein N,N'-Carbonyldiazol mit zwei identischen Azolringen. Diese zweite Durchführungsvariante ist die bevorzugte.

Bevorzugt werden weiterhin Azole eingesetzt, bei denen in den allgemeinen Formeln (I) und (II) ein oder zwei der Molekülteile X¹, X² und X³ für Stickstoff stehen. Außerdem ist es bevorzugt, dass X¹ für CH, X² für Stickstoff und X³ für CR¹ stehen, wobei R¹ und R² gemeinsam eine -CH=CH-CH=CH-Brücke bilden.

Besonders bevorzugt setzt man im erfindungsgemäßen Verfahren Imidazol, Benzimidazol, Pyrazol oder 1,2,4-Triazol als Azol der allgemeinen Formel (II) ein. Ganz besonders bevorzugt ist Imidazol.

Die genannten Azole der allgemeinen Formel (I) sind entweder käuflich erhältlich oder aber nach bekannten Verfahren des Standes der Technik herstellbar.

Es ist ein wesentliches Merkmal des erfindungsgemäßen Verfahrens, dass die gesamte Menge des umzusetzenden Azols der allgemeinen Formel (II) in dem halogenierten aliphatischen Kohlenwasserstoff vorgelegt wird und anschließend pro 1 mol Azol der allgemeinen Formel (II) 0,2 bis 0,3 mol, bevorzugt 0,22 bis 0,28 mol, besonders bevorzugt 0,25 bis 0,27 mol Phosgen eindosiert. Phosgen kann dabei in üblicher technischer Qualität eingesetzt werden.

Die Dosierung von Phosgen kann dabei kontinuierlich oder semikontinuierlich erfolgen. "Kontinuierlich" heißt dabei, dass das Phosgen ständig gleichmäßig über die gesamte Reaktionszeit in das vorgelegte Azol/Lösungsmittel-Gemisch zudosiert wird. "Semikontinuierlich" heißt, dass Phosgen portionsweise, auf definierte Zeitabschnitte verteilt in das Azol/Lösungsmittel-Gemisch eindosiert wird.

Das einzusetzende halogenierte aliphatische Lösungsmittel stammt aus der Gruppe der chlorierten aliphatischen Kohlenwasserstoffe, der bromierten aliphatischen Kohlenwasserstoffe und der gemischt Chlor-Brom-substituierten aliphatischen Kohlenwasserstoffe.

Als chlorierte aliphatische Kohlenwasserstoffe können beispielsweise Methylenchlorid, Chloroform, 1,2-Dichlorethan, 1,1-Dichlorethan oder 1,1,2,2-Tetrachlorethylen eingesetzt werden.

Als bromierte aliphatische Kohlenwasserstoffe können beispielsweise Bromoform, Dibrommethan oder 1,2-Dibromethan verwendet werden.

Weiterhin können gemischt Chlor-Brom-substituierte Kohlenwasserstoffe als Lösungsmittel verwendet werden, wie beispielsweise 1-Brom-2-chlorethan.

Der als Lösungsmittel eingesetzte halogenierte aliphatische Kohlenwasserstoff aus der oben genannten Gruppe besitzt üblicherweise einen Wassergehalt von maximal 0,5 Gew.-%, bevorzugt von maximal 0,2 Gew.-%, besonders bevorzugt von maximal 0,1 Gew.-% und insbesondere von maximal 0,05 Gew.-%.

Halogenierte aliphatische Kohlenwasserstoffe mit diesem Wassergehalt sind als Lösungsmittel entweder käuflich oder aber durch entsprechendes Andestillieren/Trocknen vor der erfindungsgemäßen Umsetzung erhältlich. Die letztere Vorgehensweise bietet sich im erfindungsgemäßen Verfahren an. Hierbei kann in zwei Varianten gearbeitet werden: Einerseits kann im Reaktionsgefäß zunächst die gesamte Menge des Lösungsmittels vorgelegt und zur Trocknung auf den gewünschten Wassergehalt andestilliert werden. Die gesamte Menge des Azols wird danach zugegeben und das Phosgen zudosiert. Andererseits kann auch die gesamte Menge des Lösungsmittels bereits zusammen mit dem Azol im Reaktionsbehältnis vorgelegt und durch Andestillieren des Azol/Lösungsmittel-Gemischs der gewünschte Wassergehalt erreicht werden, bevor das Phosgen zudosiert wird.

Das erfindungsgemäße Verfahren wird üblicherweise bei einer Temperatur im Bereich von 10 bis 100°C, vorzugsweise bei 20 bis 80°C, insbesondere bei 30 bis 65°C durchgeführt.

Im allgemeinen ist es vorteilhaft, nach dem Zudosieren von Phosgen das Reaktionsgemisch noch einige Zeit im Bereich von 30 Minuten bis 5 Stunden bei gleicher Temperatur nachzurühren.

Zur Entfernung eines eventuell vorhandenen Überschuss an Phosgen, kann die Reaktionsmischung mit Stickstoff durchgast werden, bis im Abgas kein Phosgen mehr detektierbar ist.

Ebenfalls zur Entfernung von eventuellen Überschüssen an Phosgen kann das oben beschriebene Lösungsmittel andestilliert werden, bis in der Reaktionsmischung kein Phosgen mehr detektierbar ist.

Zwecks Aufarbeitung schwemmt man das Reaktionsgemisch aus dem Reaktionsgefäß in eine Filtriervorrichtung aus. Da das Azol-Hydrochlorid auch nach Ende der Dosierung von Phosgen als kristalliner Niederschlag vorliegt, gelingt dies leicht und vollständig. Dann wird der gebildete Azol-Hydrochlorid-Niederschlag bei 10 bis 100°C, bevorzugt bei 20 bis 80°C, durch Filtration abgetrennt. Auch diese Filtration gelingt aufgrund der kristallinen Konsistenz des Niederschlages in kurzen Filtrationszeiten gut. Das N,N'-Carbonyldiazol kann aus der bei der Azol-Hydrochlorid-Abtrennung erhaltenen Mutterlauge isolieren werden, indem man die Mutterlauge auf +40 bis -70°C, bevorzugt auf +25 bis -20°C abkühlt und das dabei auskristallisierende Produkt abfiltriert. Das Produkt fällt so in gut kristallisierter Form in Reinheiten von mindestens 90 %, bevorzugt mindestens 95 % an.

Ebenso ist es möglich, die Mutterlauge nach der Azol-Hydrochlorid-Abtrennung vollständig einzuengen und damit vom Lösungsmittel zu befreien. Das so erhaltene N,N'-Carbonyldiazol besitzt ebenfalls bereits eine Reinheit von mindestens 90 %, bevorzugt von mindestens 95 %.

Die wirtschaftliche Attraktivität des erfindungsgemäßen Verfahrens läßt sich weiter erhöhen, wenn man die nach Abtrennung des auskristallisierten N,N'-Carbonyldiazols erhaltene Mutterlauge ohne weitere Aufarbeitung rezykliert und als Ausgangsbasis für eine Phosgenierung weiterer Azol-Mengen verwendet. Diese Mutterlauge kann ggf. noch Restanteile des N,N'-Carbonyldiazols enthalten. Eine derartige Rezyklierung der Mutterlauge ist mehrfach möglich. Überraschenderweise werden unverändert sehr gute Ausbeuten und hohe Reinheiten erhalten, insbesondere besitzt das unter Verwendung von rezykliertem Lösungsmittel erhaltene N,N'-Carbonyldiazol weiterhin die exzellenten Farbzahlen nach Hazen.

Eine weitere Verbesserung der wirtschaftlichen Attraktivität kann durch Rückgewinnung der Azol Menge erreicht werden, die im erfindungsgemäßen Verfahren als Azol-Hydrochlorid anfällt. Dieses Azol-Hydrochlorid kann wieder in das freie Azol überführt und somit in die Reaktion rezykliert werden. Auf diesem Weg ist es möglich, eine Verdoppelung der Ausbeute an N,N'-Carbonyldiazol, bezogen auf das eingesetzte Azol, zu erreichen.

Die Wiedergewinnung von Azolen aus Azol-Hydrochloriden kann gemäß DE-A-198 33 913 beispielsweise so durchgeführt werden, dass man die bei der Synthese der N,N'-Carbonyldiazole entstandenen Azol-Hydrochloride mit einer Verbindung der Formel (III)

M(OR⁴)ₙ (III),

in der
- n: der Wertigkeit von M entspricht,
- M: für ein Alkali- oder Erdalkalimetall steht und
- R⁴: für Wasserstoff oder C₁-C₄-Alkyl steht,
umsetzt.

Diese Umsetzung erfolgt in einem Lösungsmittelgemisch aus einerseits einem aromatischen Lösungsmittel wie beispielsweise Benzol, Toluol, einem Xylol, Monochlorbenzol, einem Dichlorbenzol, einem Trichlorbenzol oder Gemischen davon, und andererseits einem Lösungsmittel der Formel

R⁴OH (IV),

in der
- R⁴: die bei Formel (III) angegebene Bedeutung hat.

In den Formeln (III) und (IV) steht R⁴ vorzugsweise für Wasserstoff oder Methyl, in Formel (III) steht M vorzugsweise für Lithium, Natrium oder Kalium.

Es ist vorteilhaft, nach der Umsetzung des Azol-Hydrochlorids mit der Verbindung der Formel (III) die gesamte Verbindung der Formel (IV), auch die bei der Reaktion von Azol-Hydrochlorid und der Verbindung der Formel (III) entstandene Verbindung der Formel (IV) abzudestillieren, das entstandene Salz MClₙ bei normaler oder erhöhter Temperatur abzufiltrieren und das gewonnene Azol nach Abtrennung des aromatischen Lösungsmittels zur erfindungsgemäßen N,N-Carbonyl-diazol-Synthese einzusetzen.

Diese Vorgehensweise gelingt besonders gut, wenn man als Verbindung der Formel (III) LiOH, NaOH oder KOH in einem Lösungsmittelgemisch aus Wasser (das ist eine Verbindung der Formel (IV) mit R⁴ = Wasserstoff) und Chlorbenzol, Toluol, Xylol oder 2-Methyltetrahydrofuran einsetzt und das Wasser durch Azeotrop-Destillation entfernt, z.B. indem man es an einem Wasserabscheider abtrennt, oder aber, wenn man als Verbindung der Formel (III) Natriummethylat in einem Lösungsmittelgemisch aus Methanol einerseits und Chlorbenzol oder Xylol andererseits einsetzt und das Methanol durch Destillation abtrennt, z.B. indem man es aus dem Gemisch über eine wirksame Kolonne abdestilliert.

Zusammenfassend lässt sich mit dem erfindungsgemäßen Verfahren durch die Reaktion von Azol und Phosgen unter Einsatz eines halogenierten aliphatischen Kohlenwasserstoffs als Lösungsmittel ausgewählt aus der Gruppe der chlorierten aliphatischen Kohlenwasserstoffe, der bromierten aliphatischen Kohlenwasserstoffe und der gemischt Chlor- oder Brom substituierten aliphatischen Kohlenwasserstoffe, das als Nebenprodukt anfallende Azol-Hydrochlorid zuverlässig in einer nicht klebrigen Konsistenz erzeugen. Hierdurch können die Rühreigenschaften der Reaktionslösung verbessert und damit höhere Konzentrationen an Reaktanden und entsprechend höhere Raum-Zeit-Ausbeuten erzielt werden. Gleichzeitig wird die leichte Entfernung des Azol-Hydrochlorids aus dem Reaktionsgefäß sichergestellt, und Beschädigungen durch verhärtete Azol-Hydrochloride werden ausgeschlossen. Auch die Filtrationszeiten sind aufgrund des verbesserten Filtrationsverhaltens des Azol-Hydrochlorids überraschend kurz. Ein weiterer Vorteil der erfindungsgemäßen Verwendung eines halogenierten aliphatischen Lösungsmittels ist die sehr geringe Empfindlichkeit des Reaktionssystems bezüglich eventuell auftretender Phosgenüberschüsse während oder am Ende der Reaktion: Gegenüber Verfahren des Standes der Technik wird sowohl die Konsistenz des kristallinen Azol-Hydrochlorid Niederschlags als auch die Farbe des aus der Reaktionsmischung gewonnenen N,N'-Carbonyldiazols durch geringe Phosgenüberschüsse nicht oder nur minimal beeinflusst.

### Beispiele

Die Hazen-Farbzahl wird gemäß ISO 6271 bestimmt.

### Beispiel 1 (erfindungsgemäß)

In einem Kolben werden 531,5 g trockenes Dichlormethan und 93,8 g (1,37 mol) Imidazol vorgelegt und auf 35°C erhitzt. Bei dieser Temperatur werden innerhalb von 1,75 Stunden 36,04 g (0,36 mol) Phosgen mit einer Einleitungsgeschwindigkeit von 20,6 g/h zugegeben. Die so erhaltene Mischung wird noch 1,5 h bei gleicher Temperatur nachgerührt.

Um eine phosgenfreie Reaktionsmischung sicherzustellen, werden bei einem Druck von 790 bis 500 mbar und 35-25°C 13,2 g Destillat abgenommen, das mit einem Ammoniak-Wasser-Isopropanol-Gemisch versetzt und verworfen wird.

Das als Nebenprodukt entstehende Imidazol-Hydrochlorid (isoliertes Trockengewicht: 72,1 g) wird durch Filtration bei 35°C entfernt, wobei der Filterkuchen mit zweimal je 100 ml 33°C warmen Dichlormethan gewaschen wird.

Von den vereinigten organischen Phasen werden bei 790 bis 500 mbar und 35-25°C 250,1 g wasserklare Lösung abdestilliert. Die zurückbleibende Lösung wird auf 0°C gekühlt, wobei eine Suspension entsteht. Das ausgefallene Carbonylbisimidazol wird durch Filtration abgetrennt und nochmals mit 50 ml auf 0°C temperiertem Dichlormethan gewaschen.

Nach Trocknung der Kristalle bei 4 mbar und 30°C werden 40,0 g Produkt in Form weißer Kristalle, Hazen-Farbzahl: 69,7 erhalten. Die Reinheit des Produkts beträgt 99,3 %, entsprechend einer Ausbeute von 71,2 % der Theorie.

**Beispiel 2 (Vergleich analog zu Beispiel 1 aus** WO-A-00/14072)

In einen Kolben werden 68,22 g Imidazol in 505 g Xylol suspendiert. Die Mischung wird bis zum Rückfluss erhitzt, wobei zur Entwässerung 5 g eines Xylol/Wasser-Gemischs abgenommen werden. Die Temperatur wird auf 66°C reduziert und innerhalb von 30 min werden 25,2 g Phosgen mit einer Einleitgeschwindigkeit von 50,4 g/h zudosiert.

Nach etwa 15 min nimmt die Reaktionsmischung dabei eine kaugummiartige Konsistenz an. Nach beendeter Phosgendosierung liegt als Nebenprodukt das Imidazol-Hydrochlorid in Form von gelben Kugeln vor. Nachdem eine weitere Stunde bei dieser Temperatur gerührt wird, wird diese Temperatur auf 130°C angehoben, wobei sich die Konsistenz des Imidazol-Hydrochlorids zu einer braunen Schmelze verändert.

Bei 130°C wird die Schmelze abgelassen. Diese erstarrt beim Abkühlen zu einer dunkelgrünen festen Masse.

Die überstehende Xylol-Phase wird auf 0°C gekühlt. Die ausgefallenen Kristalle werden abfiltriert und bei 20 mbar und 50°C getrocknet.

Man erhält Carbonylbisimidazol in Form von weißen Kristallen mit schwarzen Anteilen (Hazen-Farbzahl: 489). Die Reinheit beträgt 96,8 %, entsprechend einer Ausbeute von 70 % der Theorie.

### Beispiel 3 (erfindungsgemäß)

In einem Kolben werden 375,2 g trockenes Chloroform und 93,8 g Imidazol vorgelegt und auf 35°C erhitzt. Bei dieser Temperatur werden innerhalb von 1,75 Stunden 35,02 g Phosgen mit einer Einleitgeschwindigkeit von 20,0 g/h zugegeben. Die so erhaltene Mischung wird noch 1,5 h bei gleicher Temperatur nachgerührt.

Um eine phosgenfreie Reaktionsmischung sicherzustellen, werden bei einem Druck von 280 mbar und 30°C 28 g Destillat abgenommen, das mit einem Ammoniak-Wasser-Isopropanol-Gemisch versetzt und verworfen wird.

Das als Nebenprodukt entstehende Imidazol-Hydrochlorid (isoliertes Trockengewicht: 72,1 g) wird durch Filtration bei 35°C entfernt, wobei der Filterkuchen mit zweimal je 100 ml 35°C warmen Chloroform gewaschen wird.

Von den vereinigten organischen Phasen werden bei 280 mbar und 30°C 251,1 g wasserklare Lösung abdestilliert. Die zurückbleibende Lösung wird auf 0°C gekühlt, wobei eine Suspension entsteht. Das ausgefallene Carbonylbisimidazol wird durch Filtration abgetrennt und nochmals mit 50 ml auf 0°C temperiertem Chloroform gewaschen.

Nach Trocknung der Kristalle bei 6 mbar und 30°C werden 41,5 g Produkt in Form weißer Kristalle mit einer Hazen-Farbzahl von 44 erhalten. Die Reinheit des Produkts beträgt 99,5 %, entsprechend einer Ausbeute von 74,0 % der Theorie.

### Beispiel 4 (erfindungsgemäß)

In einem Kolben werden 358,1 g trockenes Chloroform und 119,36 g Imidazol vorgelegt und auf 55°C erhitzt. Bei dieser Temperatur werden innerhalb von 1,75 Stunden 44,57 g Phosgen mit einer Einleitgeschwindigkeit von 25,5 g/h zugegeben. Die so erhaltene Mischung wird noch 2 h bei gleicher Temperatur nachgerührt.

Um eine phosgenfreie Reaktionsmischung sicherzustellen, werden bei einem Druck von 630 mbar und 35°C 5,4 g Destillat abgenommen, das mit einem Ammoniak-Wasser-Isopropanol-Gemisch versetzt und verworfen wird.

Das als Nebenprodukt entstehende Imidazolhydrochlorid (isoliertes Trockengewicht: 96,0 g) wird durch Filtration bei 55°C entfernt, wobei der Filterkuchen mit zweimal je 100 ml 55°C warmen Chloroform gewaschen wird.

Die vereinigten organischen Phasen werden auf 0°C gekühlt, wobei eine Suspension entsteht. Das ausgefallene Carbonylbisimidazol wird durch Filtration abgetrennt und nochmals mit 50 ml auf 0°C temperiertem Chloroform gewaschen.

Nach Trocknung des Rückstands bei 4 mbar und 46°C werden 44,3 g Produkt in kristalliner Form mit einer Hazen-Farbzahl von 49 erhalten. Die Reinheit des Produkts beträgt 99,0 %, entsprechend einer Ausbeute von 61,9 % der Theorie.

### Beispiel 5 (erfindungsgemäß unter Rezyklierung der Mutterlauge)

### 1. Phosgenierung

In einem Kolben werden 531,5 g trockenes Dichlormethan und 93,8 g (1,37 mol) Imidazol vorgelegt und auf 35°C erhitzt. Bei dieser Temperatur werden innerhalb von 1,75 Stunden 35,02 g (0,35 mol) Phosgen mit einer Einleitungsgeschwindigkeit von 20,0 g/h zugegeben. Die so erhaltene Mischung wird noch 1,5 h bei gleicher Temperatur nachgerührt.

Um eine phosgenfreie Reaktionsmischung sicherzustellen, werden bei einem Druck von 750 bis 500 mbar und 35-20°C 8,4 g Destillat abgenommen, das mit einem Ammoniak-Wasser-Isopropanol-Gemisch versetzt und verworfen wird.

Das als Nebenprodukt entstehende Imidazol-Hydrochlorid (isoliertes Trockengewicht: 80,3 g) wird durch Filtration bei 35°C entfernt, wobei der Filterkuchen mit zweimal je 100 ml 33°C warmen Dichlormethan gewaschen wird.

Die zurückbleibende Lösung wird auf 0°C gekühlt, wobei eine Suspension entsteht. Das ausgefallene Carbonylbisimidazol wird durch Filtration abgetrennt und nochmals mit 50 ml auf 0°C temperiertem Dichlormethan gewaschen. Nach Abfiltrieren des festen Carbonylbisimidazol fallen 553,0 g Mutterlauge M1 an.

Nach Trocknung der Kristalle bei 5 mbar und 20°C werden 33,54 g Produkt in Form weißer Kristalle mit einer Hazen-Farbzahl von 45,1 erhalten. Die Reinheit des Produkts beträgt 99,6 %. Die Ausbeute entspricht somit 59,9 % der Theorie.

### 2. Phosgenierung

In einem Kolben werden 531,5 g Dichlormethan-enthaltende Mutterlauge M1 aus dem 1. Phosgenierungsschritt und 93,8 g (1,37 mol) Imidazol vorgelegt und auf 35°C erhitzt. Bei dieser Temperatur werden innerhalb von 1,75 Stunden 35,02 g (0,35 mol) Phosgen mit einer Einleitungsgeschwindigkeit von 20,0 g/h zugegeben. Die so erhaltene Mischung wird noch 1,5 h bei gleicher Temperatur nachgerührt.

Um eine phosgenfreie Reaktionsmischung sicherzustellen, werden bei einem Druck von 750 bis 450 mbar und 35-20°C 21,6 g Destillat abgenommen, das mit einem Ammoniak-Wasser-Isopropanol-Gemisch versetzt und verworfen wird.

Das als Nebenprodukt entstehende Imidazol-Hydrochlorid (isoliertes Trockengewicht: 86,6 g) wird durch Filtration bei 35°C entfernt, wobei der Filterkuchen mit zweimal je 100 ml 33°C warmen Dichlormethan gewaschen wird.

Die zurückbleibende Lösung wird auf 0°C gekühlt, wobei eine Suspension entsteht. Das ausgefallene Carbonylbisimidazol wird durch Filtration abgetrennt und nochmals mit 100 ml auf 0°C temperiertem Dichlormethan gewaschen. Nach Abfiltrieren des festen Carbonylbisimidazol fallen 553,0 g Mutterlauge M2 an.

Nach Trocknung der Kristalle bei 6 mbar und 30°C werden 39,4 g Produkt in Form weißer Kristalle mit einer Hazen-Farbzahl von 33,2 erhalten. Die Reinheit des Produkts beträgt 98,7 %. Die Ausbeute entspricht somit 70 % der Theorie.

### 3. Phosgenierung

In einem Kolben werden 531,5 g Dichlormethan enthaltende Mutterlauge M2 aus dem 2. Phosgenierungsschritt und 93,8 g (1,37 mol) Imidazol vorgelegt und auf 35°C erhitzt. Bei dieser Temperatur werden innerhalb von 1,75 Stunden 35,02 g (0,35 mol) Phosgen mit einer Einleitungsgeschwindigkeit von 20,0 g/h zugegeben. Die so erhaltene Mischung wird noch 1,5 h bei gleicher Temperatur nachgerührt.

Um eine phosgenfreie Reaktionsmischung sicherzustellen, werden bei einem Druck von 750 bis 500 mbar und 35-20°C 9,2 g Destillat abgenommen, das mit einem Ammoniak-Wasser-Isopropanol-Gemisch versetzt und verworfen wird.

Das als Nebenprodukt entstehende Imidazol-Hydrochlorid (isoliertes Trockengewicht: 88,1 g) wird durch Filtration bei 35°C entfernt, wobei der Filterkuchen mit zweimal je 100 ml 33°C warmen Dichlormethan gewaschen wird.

Die zurückbleibende Lösung wird auf 0°C gekühlt, wobei eine Suspension entsteht. Das ausgefallene Carbonylbisimidazol wird durch Filtration abgetrennt und nochmals mit 100 ml auf 0°C temperiertem Dichlormethan gewaschen.

Nach Trocknung der Kristalle bei 7 mbar und 20°C werden 37,3 g Produkt in Form weißer Kristalle mit einer Hazen-Farbzahl von 41,0 erhalten. Die Reinheit des Produkts beträgt 98,5 %. Die Ausbeute entspricht somit 65,9 % der Theorie.

## Patentansprüche

1. Verfahren zur Herstellung von N,N'-Carbonyldiazolen der allgemeinen Formel (I) 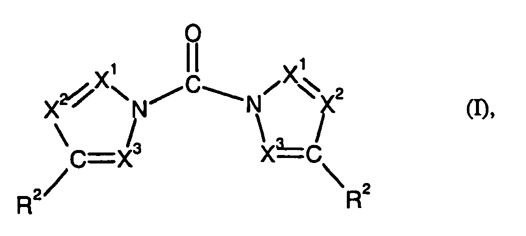 wobei entweder
X¹, X² und X³ unabhängig voneinander jeweils für CR¹ oder Stickstoff stehen, wobei R¹ Wasserstoff oder geradkettiges oder verzweigtes C₁-C₆-Alkyl bedeutet und
R² Wasserstoff bedeutet,
oder
X¹ und X³ für CR¹ stehen, wobei der in X¹ befindliche Rest R¹ Wasserstoff oder geradkettiges oder verzweigtes C₁-C₆-Alkyl bedeutet und der in X³ befindliche Rest R¹ gemeinsam mit R² eine -CH=CH-CH=CH-Brücke bildet, und
X² für CR¹ oder Stickstoff steht, wobei R¹ Wasserstoff oder geradkettiges oder verzweigtes C₁-C₆-Alkyl bedeutet,
durch Umsetzung von Azolen der allgemeinen Formel (II), 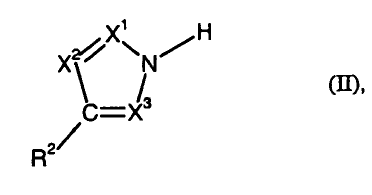 in der die verwendeten Reste und Symbole die für die allgemeine Formel (I) angegebenen Bedeutungen haben,
mit Phosgen, **dadurch gekennzeichnet ist, dass**
(i) ein halogeniertes aliphatisches Lösungsmittel aus der Gruppe der chlorierten aliphatischen Kohlenwasserstoffe, der bromierten aliphatischen Kohlenwasserstoffe und der gemischt Chlor-Brom-substituierten aliphatischen Kohlenwasserstoffe verwendet wird und
(ii) dass man die gesamte umzusetzende Menge Azol vorlegt und anschließend Phosgen eindosiert.

2. Verfahren nach Anspruch 1, **dadurch gekennzeichnet, dass** entweder zwei verschiedene oder aber auch nur ein einziges Azol der allgemeinen Formel (II) eingesetzt werden.

3. Verfahren nach Anspruch 1 oder 2, **dadurch gekennzeichnet, dass** ein oder zwei Azole der allgemeinen Formel (II) eingesetzt werden, bei denen unabhängig voneinander ein oder zwei der Molekülteile X¹, X² und X³ für Stickstoff stehen.

4. Verfahren nach einem oder mehreren der Ansprüche 1 bis 3, **dadurch gekennzeichnet, dass** ein oder zwei Azole der allgemeinen Formel (II) eingesetzt wird, bei denen unabhängig voneinander X¹ für CH, X² für Stickstoff und X³ für CR¹ stehen, wobei R¹ und R² gemeinsam eine -CH=CH-CH=CH-Brücke bilden.

5. Verfahren nach einem oder mehreren der Ansprüche 1 bis 4, **dadurch gekennzeichnet, dass** Imidazol, Benzimidazol, Pyrazol oder 1,2,4-Triazol als Azol der allgemeinen Formel (II) eingesetzt werden.

6. Verfahren nach einem oder mehreren der Ansprüche 1 bis 5, **dadurch gekennzeichnet, dass** insgesamt pro mol Azol der allgemeinen Formel (II) 0,2 bis 0,3 mol, Phosgen, eingesetzt werden.

7. Verfahren nach einem oder mehreren der Ansprüche 1 bis 6, **dadurch gekennzeichnet, dass** als chlorierte aliphatische Kohlenwasserstoffe Methylenchlorid, Chloroform, 1,2-Dichlorethan, 1,1-Dichlorethan und 1,1,2,2-Tetrachlorethylen eingesetzt werden.

8. Verfahren nach einem oder mehreren der Ansprüche 1 bis 6, **dadurch gekennzeichnet, dass** als bromierte aliphatische Kohlenwasserstoffe Bromoform, Dibrommethan oder 1,2-Dibromethan eingesetzt werden.

9. Verfahren nach einem oder mehreren der Ansprüche 1 bis 6, **dadurch gekennzeichnet, dass** als gemischt Chlor-Brom substituierter Kohlenwasserstoff 1-Brom-2-chlorethan eingesetzt wird.

10. Verfahren nach einem oder mehreren der Ansprüche 1 bis 9, **dadurch gekennzeichnet, dass** das Lösungsmittel einen Wassergehalt von maximal 0,5 Gew.-%, besitzt.

11. Verfahren nach einem oder mehreren der Ansprüche 1 bis 9, **dadurch gekennzeichnet, dass** man das verwendete Lösungsmittel gegebenenfalls in Gegenwart des Azols durch Andestillieren trocknet.

12. Verfahren nach einem oder mehreren der Ansprüche 1 bis 11, **dadurch gekennzeichnet, dass** man das Reaktionsgemisch so aufarbeitet, dass man den gebildeten Azol-Hydrochlorid-Niederschlag bei 10 bis 100°C durch Filtration abtrennt und aus dem Filtrat N,N'-Carbonyldiazol isoliert, indem man die Mutterlauge auf +40 bis -70°C abkühlt und das dabei auskristallisierende N, N'-Carbonyldiazol abfiltriert.

13. Verfahren nach Anspruch 12, **dadurch gekennzeichnet, dass** man die nach Abtrennung des N,N'-Carbonyldiazols erhaltene Mutterlauge ohne weitere Aufarbeitung rezykliert und für eine Phosgenierung weiterer Mengen eines Azols der allgemeinen Formel (II) verwendet.

14. Verfahren nach einem oder mehreren der Ansprüche 1 bis 12, **dadurch gekennzeichnet, dass** man das Reaktionsgemisch nach der Phosgenierung so aufarbeitet, dass man den gebildeten Azol-Hydrochlorid-Niederschlag bei 20 bis 80°C wie in Anspruch 12 angegeben durch Filtration abtrennt und das Filtrat vollständig einengt und damit das Lösungsmittel entfernt.

## Claims

1. Process for preparing N,N'-carbonyldiazoles of the general formula (I) 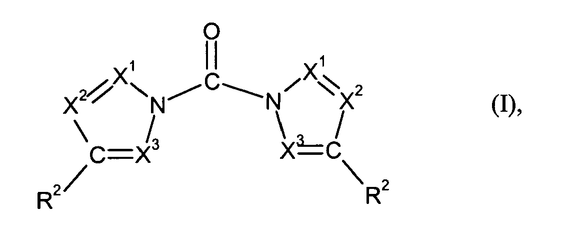 where either
X¹, X² and X³ independently of one another are each CR¹ or nitrogen, R¹ being hydrogen or straight-chain or branched C₁-C₆ alkyl, and
R² is hydrogen,
or
X¹ and X³ are CR¹, the radical R¹ in X¹ being hydrogen or straight-chain or branched C₁-C₆ alkyl and the radical R¹ in X³ forming, together with R², a -CH=CH-CH=CH- bridge, and
X² is CR¹ or nitrogen, R¹ being hydrogen or straight-chain or branched C₁-C₆ alkyl,
by reacting azoles of the general formula (II), 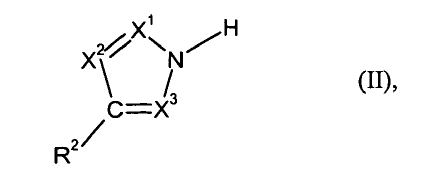 in which the radicals and symbols used have the definitions indicated for the general formula (I),
with phosgene, this process being **characterized in that**
(i) a halogenated aliphatic solvent from the group consisting of chlorinated aliphatic hydrocarbons, brominated aliphatic hydrocarbons and mixedly chlorine-/bromine-substituted aliphatic hydrocarbons is used and
(ii) **in that** the entire amount of azole to be reacted is introduced as an initial charge and then phosgene is metered in.

2. Process according to Claim 1, **characterized in that** either two different azoles or else only one single azole of the general formula (II) are or is used.

3. Process according to Claim 1 or 2, **characterized in that** one or two azoles of the general formula (II) is or are used in which independently of one another one or two of the moieties X¹, X² and X³ is or are nitrogen.

4. Process according to one or more of Claims 1 to 3, **characterized in that** one or two azoles of the general formula (II) is or are used in which independently of one another X¹ is CH, X² is nitrogen and X³ is CR¹, R¹ and R² together forming a -CH=CH-CH=CH- bridge.

5. Process according to one or more of Claims 1 to 4, **characterized in that** imidazole, benzimidazole, pyrazole or 1,2,4-triazole is used as the azole of the general formula (II).

6. Process according to one or more of Claims 1 to 5, **characterized in that** in total 0.2 to 0.3 mol of phosgene is used per mole of azole of the general formula (II).

7. Process according to one or more of Claims 1 to 6, **characterized in that** chlorinated aliphatic hydrocarbons used are methylene chloride, chloroform, 1,2-dichloroethane, 1,1-dichloroethane and 1,1,2,2-tetrachloroethylene.

8. Process according to one of more of Claims 1 to 6, **characterized in that** brominated aliphatic hydrocarbons used are bromoform, dibromomethane or 1,2-dibromoethane.

9. Process according to one or more of Claims 1 to 6, **characterized in that** a mixedly chlorine-/bromine-substituted hydrocarbon used is 1-bromo-2-chloroethane.

10. Process according to one or more of Claims 1 to 9, **characterized in that** the solvent possesses a water content of not more than 0.5% by weight.

11. Process according to one or more of Claims 1 to 9, **characterized in that** the solvent used is dried by partial distillation optionally in the presence of the azole.

12. Process according to one or more of Claims 1 to 11, **characterized in that** the reaction mixture is worked up by separating off the azole hydrochloride precipitate at 10 to 100°C by filtration and isolating N,N'-carbonyldiazole from the filtrate by cooling the mother liquor to +40 to -70°C and filtering off the N,N'-carbonyldiazole that crystallizes out in the course of cooling.

13. Process according to Claim 12, **characterized in that** the mother liquor obtained after the N,N'-carbonyldiazole has been separated off is recycled without further working-up and is used for the phosgenation of further quantities of an azole of the general formula (II).

14. Process according to one or more of Claims 1 to 12, **characterized in that** after the phosgenation the reaction mixture is worked up by separating off the azole hydrochloride precipitate at 20 to 80°C by filtration as indicated in Claim 12 and concentrating the filtrate completely and thereby removing the solvent.

## Revendications

1. Procédé de fabrication de N,N'-carbonyldiazoles de formule générale (I) 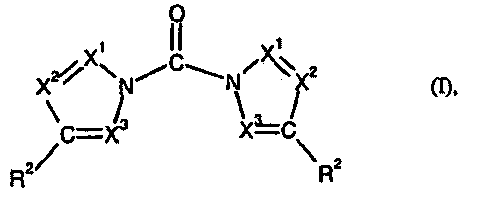 dans laquelle :
X¹, X² et X³ représentent respectivement, indépendamment les uns des autres, un groupement CR¹ ou l'azote, R¹ représentant l'hydrogène ou un groupement alkyle en C₁-C₆ linéaire ou ramifié, et
R² représente l'hydrogène, ou
X¹ et X³ représentent un groupement CR¹, le radical R¹ que l'on trouve dans le radical X¹ représentant l'hydrogène ou un groupement alkyle en C₁-C₆ linéaire ou ramifié et le radical R¹ que l'on trouve dans le radical X³ formant conjointement avec R² un pont -CH=CH-CH=CH-, et
X² représente un groupement CR¹ ou l'azote, R¹ représentant l'hydrogène ou un groupement alkyle en C₁-C₆ linéaire ou ramifié,
en faisant réagir des azoles de formule générale (II) 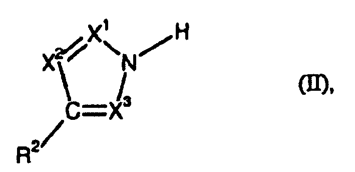 dans laquelle les radicaux et les symboles utilisés ont les significations données pour la formule générale (I),
avec du phosgène, **caractérisé en ce que** :
(i) on utilise un solvant aliphatique halogéné choisi dans le groupe des hydrocarbures aliphatiques chlorés, des hydrocarbures aliphatiques bromés et des hydrocarbures aliphatiques à substitution mixte chlore-brome, et
(ii) on incorpore la quantité totale d'azole que l'on souhaite faire réagir, puis on ajoute le phosgène de manière dosée.

2. Procédé selon la revendication 1, **caractérisé en ce que** l'on utilise soit deux azoles différents soit, également, un seul azole de formule générale (II).

3. Procédé selon la revendication 1 ou 2, **caractérisé en ce que** l'on utilise un ou deux azoles de formule générale (II), dans lesquels une ou deux des parties de molécule X¹, X² et X³ représentent, indépendamment l'une de l'autre, l'azote.

4. Procédé selon une ou plusieurs des revendications 1 à 3, **caractérisé en ce que** l'on utilise un ou deux azoles de formule générale (II), dans lesquels, indépendamment les uns des autres, X¹ représente CH, X² représente l'azote et X³ représente un groupement CR¹, R¹ et R² formant conjointement un pont -CH=CH-CH=CH-.

5. Procédé selon une ou plusieurs des revendications 1 à 4, **caractérisé en ce que** l'on utilise comme azole de formule générale (II), l'imidazole, le benzimidazole, le pyrazole ou le 1,2,4-triazole.

6. Procédé selon une ou plusieurs des revendications 1 à 5, **caractérisé en ce que** l'on utilise, au total, 0,2 à 0,3 mole de phosgène par mole d'azole de formule générale (II).

7. Procédé selon une ou plusieurs des revendications 1 à 6, **caractérisé en ce que** l'on utilise comme hydrocarbures aliphatiques chlorés, le chlorure de méthylène, le chloroforme, le 1,2-dichloroéthane, le 1,1-dichloroéthane et le 1,1,2,2-tétrachloroéthylène.

8. Procédé selon une ou plusieurs des revendications 1 à 6, **caractérisé en ce que** l'on utilise comme hydrocarbures aliphatiques bromés, le bromoforme, le dibromométhane ou le 1,2-dibromoéthane.

9. Procédé selon une ou plusieurs des revendications 1 à 6, **caractérisé en ce que** l'on utilise comme hydrocarbure à substitution mixte chlore-brome, le 1-bromo-2-chloroéthane.

10. Procédé selon une ou plusieurs des revendications 1 à 9, **caractérisé en ce que** le solvant possède une teneur en eau de 0,5 % en poids au maximum.

11. Procédé selon une ou plusieurs des revendications 1 à 9, **caractérisé en ce que** l'on sèche le solvant utilisé, éventuellement en présence de l'azole, en le séparant par distillation.

12. Procédé selon une ou plusieurs des revendications 1 à 11, **caractérisé en ce que** l'on traite le mélange réactionnel de manière à séparer par filtration le précipité de chlorhydrate d'azole formé, à une température dans la plage de 10 à 100 °C, et **en ce que** l'on isole le N,N'-carbonyldiazole du substrat en refroidissant la lessive mère à une température dans la plage de +40 à -70 °C et en séparant par filtration le N,N'-carbonyldiazole lequel, en l'occurrence, recristallise.

13. Procédé selon la revendication 12, **caractérisé en ce que** l'on recycle la lessive mère obtenue après que l'on a séparé le N,N'-carbonyldiazole, sans effectuer de traitement supplémentaire, et **en ce que** l'on utilise d'autres quantités d'un azole de formule générale (II) pour réaliser une réaction de phosgénation.

14. Procédé selon une ou plusieurs des revendications 1 à 12, **caractérisé en ce que** l'on traite le mélange réactionnel après phosgénation de manière à séparer par filtration le précipité de chlorhydrate d'azole formé, à une température dans la plage de 20 à 80 °C, comme indiqué dans la revendication 12, et **en ce que** l'on concentre totalement le filtrat, éliminant de la sorte le solvant.
